# EUROPEAN PATENT SPECIFICATION

(11) **EP 0 540 719 B1**
(45) Date of publication and mention of the grant of the patent: **13.01.1999**
(21) Application number: 92911076.5
(22) Date of filing: 18.05.1992
(51) Int. Cl.: C12N 15/80, C12N 15/30, C12N 1/15, C12N 15/52, C12N 9/00, C07K 14/00

(54) **DICTYOSTELID EXPRESSION VECTOR AND METHOD FOR EXPRESSING A DESIRED PROTEIN**
DYCTOSTELIDER EXPRESSIONSVEKTOR UND VERFAHREN ZUR EXPRESSION VON EINEM GEWÜNSCHTEN PROTEIN
VECTEUR D'EXPRESSION DE DICTYOSTELIDES ET PROCEDE D'EXPRESSION D'UNE PROTEINE DESIREE

(30) Priority: 17.05.1991 NL 9100869
(43) Date of publication of application: 12.05.1993
(73) Proprietor: RMF DICTAGENE S.A., 1008 Prilly (CH)
(72) Inventor: FASEL, Nicolas, Joseph, CH-1066 Epalinges (CH); REYMOND, Christophe, Dominique, CH-1008 Prilly (CH)
(74) Representative: Bruin, Cornelis Willem
(86) International application number: EP9201112
(87) International publication number: WO9220806

(56) References cited:
- EP-A- 278 941
- WO-A-91/06644
- Gene, vol. 85, 1989, Elsevier Science Publishers B.V., (Amsterdam, NL), T. DINGERMANN et al.: "Optimization and in situ detection of Escherichia coli beta-galactosidase gene expression in Dictyostelium discoideum", page 353-362, see the whole article
- Nucleic Acids Research, vol. 18, no. 18, 1990, Oxford University Press, (Arlington, US), M. MANIAK et al.: "Evidence for a feedback regulated back-up promoter which controls permanent expression of a Dictyostelium gene", pages 5375-5380, see page 5376, left-hand column, paragraphs 3-4; figure 1
- Proc. Natl. Acad. Sci. USA, vol. 82, October 1985, Genetics, (Washington, DC, US), C.D. REYMOND et al.: "Regulated expression of ras gene constructs in Dictyostelium transformants", pages 7005-7009, see the whole document
- Biochem. Cell. Biology, vol. 66, no. 10, 10 October 1988, C.-H. SIU et al.: "Molecular mechanisms of cell-cell interaction in Dictyostelium discoideum", pages 1089-1099, see page 1094, left-hand column, paragraph 3; figure 5, line 1
- Molecular and Cellular Biology, vol. 10, no. 8, August 1990, American Soc. for Microbiology, (Washington, DC, US), F. VAUTI et al.: "Regulation of the discoidin I gamma gene in Dictyostelium discoideum: identification of individual promoter elements mediating induction of transcription and repression by cyclic AMP", pages 4080-4088, see the whole article
- Gene, vol. 111, 15 February 1992, Elsevier Science Publishers B.V., (Amsterdam, NL), N. FASEL et al.: "Dictyostelium discoideum as an expression host for the circumsporozoite protein of Plasmodium falciparum", pages 157-163, see the whole article

## Description

The present invention relates to recombinant DNA molecules and a method for expressing desired DNA sequences in dictyostelids. More in particular it relates to a recombinant plasmid and method for expressing a desired enzyme or parasite antigen in dictyostelids and more in particular to expressing the circumsporozoite antigen of *Plasmodium falciparum* and chloramphenicol acetyl transferase in *Dictyostelium discoideum.*

Production of functional or immunogenic recombinant polypeptides has been obtained in multiple organisms. In general, the gen of interest has been isolated and expressed under the control of specific DNA elements allowing expression in the particular host. Bacteria, lower and higher eukaryotic cells and viral vectors have been among the most widely used systems.

*Plasmodium falciparum*, the most frequent human malaria causative parasite, is found in different forms in insect and human hosts. The use of inactivated parasite forms as vaccine in mammals have shown promising results. A limitation, however, being the low amount of material available due to the difficulty of cultivating parasites. The genes coding for some cell surface proteins of the different forms have been sequenced allowing to identify host protective antigens possibly useful as vaccines. Expression of such antigens or other *P. falciparum* proteins in heterologous recombinant systems (e.g. E. coli, yeast, vaccinia virus, baculovirus, salmonella) has been difficult, and in most instances, only small amounts of complete proteins were obtained.

The surface of the first form of *Plasmodium falciparum* found in the organism after insect bite transmission, is covered by the circumsporozoite (CS) protein. This protein is synthesized in the form of a polypeptide precursor which is composed of an amino terminal signal sequence removed upon processing, of a large central repeat domain flanked on both sides by regions referred as region I and II containing conserved sequences between different *Plasmodia* species and of a terminal carboxy anchor domain. The repeat domain consisting of (ASN-ALA-ASN-PRO)ₙ has been shown to be the B-cell immunodominant region of the *P. falciparum* CS. Synthetic peptides containing such a repeat have been used with limited success as subunit vaccine in protection studies. The T-cell response elements on the CS protein have been mapped outside of the repeat segments. These results suggest that the entire CS protein could be used to obtain a stronger and longer lasting immune protection. These experimental data further indicate the requirement for a protein with its B- and T-cell epitopes to obtain an efficient malaria vaccine.

Difficulties in producing high amounts of CS protein were bypassed by expressing only segments of the CS protein in vivo which, however, did not elicit a sufficient immune response as vaccines. Expression of the complete protein was obtained using vaccinia and baculovirus expression systems. However, unstable expression of proteins as well as the high cost of production render such systems unattractive.

It is therefore an object of the present invention to provide an expression system for different kinds of proteins, such as *Plasmodium falciparum* proteins, that is capable of stable expression of proteins at low cost.

It has been found that species of the slime mold *Dictyostelium* can be used as an efficient eukaryotic expression system for the production of recombinant proteins.

The cellular slime mold *Dictyostelium discoideum* (Dd) is a free-living organism, easy to grow and to maintain. Dd strains can grow on bacteria lawns with a doubling time of about 3 hours, in bacterial suspensions to high densities (up to 10¹⁰ cells per liter) or in semi-synthetic media containing glucose, peptone and yeast extract where doubling time is about 12 hours. The life cycle of *Dictyostelium* consists of a growth and a developmental phase. The developmental phase is triggered by starvation and is characterized by aggregation of previously single cells to form a multicellular organism which then differentiates to produce spores which can be stored over a prolonged period of time. Germination of spores in the presence of bacteria or rich medium will allow renewed growth. During this developmental cycle, diffusible factors are produced and for at least one of them (cAMP) binding to its receptor induces transcription of a set of specific genes (See Loomis, The development of *Dictyostelium discoideum,* Acad. Press, 1982).

Growth properties and transformation capacity of *Dictyostelium discoideum* offers the possibility to express foreign proteins, since cells can be grown at low cost on bacteria and expression of specific proteins can be tightly controlled by starvation in a simple medium.

An expression system for Dictyostelium discoideum has been described by Dingermann et al. in Gene 85, 353-362 (1989). Although expression of β-galactosidase has been found by the authors, it was found by the present inventors that the expression system described is not suitable for obtaining high yields of in particular functional parasite proteins.

The present invention therefore provides an expression system with which high yields of functional proteins can be obtained, which expression system consists of a recombinant DNA molecule for expression of a desired functional polypeptide or intermediate thereof in dictyostelid hosts, comprising:
a) a Dictyostelium discoideum homologous promoter region;
b) a Dictyostelium discoideum homologous terminator region;
c) a heterologous DNA sequence with a Dictyostelium discoideum homologous peptide sequence being capable of functioning as a leader peptide sequence positioned upstream thereof and in proper reading frame therewith,
wherein the heterologous DNA sequence encodes the desired functional polypeptide or intermediate thereof, said DNA sequence being positioned downstream from the promoter region and said terminator region being positioned downstream from the DNA sequence, wherein the Dictyostelium discoideum homologous leader peptide sequence is a sequence other than the D. discoideum PsA protein leader peptide sequence.

In one embodiment of this invention the homologous promoter region is the Discoidin I promoter of *Dictyostelium discoideum* which is under developmental control. Transcription from this promoter is induced by starvation of the *Dictyostelium* cell culture.

Furthermore a leader peptide sequence is provided in the expression vector. Fusion of this leader peptide allows export of the recombinant protein to the cell surface thus facilitating recombinant protein identification.

In another embodiment the invention provides an inducible expression vector. The Dd ras promoter of *Dictyostelium discoideum* is used there as the homologous promoter region. Expression of genes under the control of the Dd ras promoter is triggered by cAMP addition to the cell culture. During cell growth the transcription level from the Dd ras promoter is undetectable, thus allowing to introduce genes encoding potentially toxic proteins in *Dictyostelium.* The production of these proteins is triggered by cAMP addition during development.

The invention is further illustrated by the following examples, which should not be considered as limiting the scope of the present invention.

### EXAMPLES

The following examples utilize many techniques well known and accessible to those skilled in the art of molecular biology. Such methods are not always described in detail.

Enzymes are obtained from commercial sources and used according to the supplier's protocols.

Bacterial media and current cloning techniques are described in Sambrook et al. (Molecular cloning: A laboratory manual, CSH Press, 1989.)

Monoclonal antibodies and NANP₅₀ peptide were obtained from H. Matile (Hoffman La Roche Ltd.).

### EXAMPLE 1

The following examples teach the expression of the circumsporozoite antigen CS of *Plasmodium falciparum* in *Dictyostelium discoideum* under the control of the Discoidin I promoter.

### 1.1. Construction of CS containing plasmids.

Expression vector pEDI-CS is constituted of the pVEII vector (Maniak and Nellen, Nucl. Acids Res., 18, 5375, 1990), which contains the elements important for propagation and maintenance in a prokaryotic host (origin of replication and ampicillin resistance), and of a Tn903 encoded neomycin resistance gene conferring geneticin (G418) resistance to eukaryotic cells under the control of a *Dictyostelium* actin 15 transcription unit. The presence of a Discoidin I promoter allows the developmental control of expression of downstream sequences and actin 8 sequences insure proper termination of the RNA.

For construction of the pEDI-CS expression vector the HaeIII + RsaI restriction fragment of 1161 bp of the CS NF54 gene (Caspers et al., Mol. and Biochem. Parasitol 35, 185, 1989) was first inserted onto the Asp718+BamHI site of pVEII filled in by Klenow DNA polymerase.

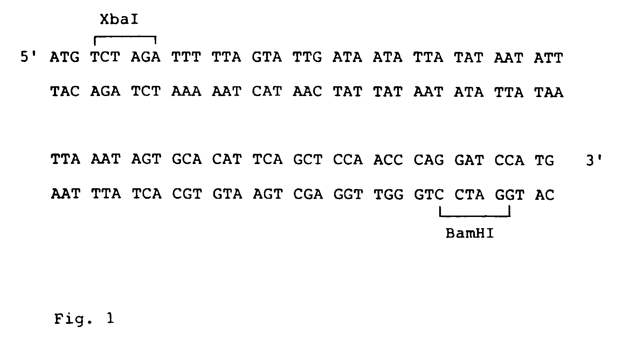

Subsequently both DNA strands of a sequence encoding the contact site A (CsA) leader peptide plus 3 amino acids were synthesized on a Applied Biosystem Model 380 B DNA synthesizer. The nucleotide sequence of the synthetic leader peptide (Fig. 1) was confirmed by introducing the blunt end fragment onto M13mp18 replicative form at the SmaI site, followed by DNA sequencing.

The XbaI+BamHI restriction fragment containing CsA leader peptide was then isolated and inserted at the XbaI-+Bam HI sites present in the vector to generate expression vector pEDII-CS.

### 1.2. Transformation of Dictyostelium discoideum.

*Dictyostelium* cells were cultured in shaking suspensions in HL-5 medium up to a concentration of 2-5x10⁶/ml, centrifuged at 300g and rinsed in distilled water. 5 µg of the desired DNA was electroporated onto 10⁷ cells in 100 µl of distilled water at 670 V and 3 µF using a Gene Pulser apparatus from BioRad, thus delivering a pulse of about 1.5 msec.

Progressive G418 selection was applied to the cells up to a concentration of 50 µg/ml, ensuring the presence of 100 to 200 copies of tandem repeats of inserted DNA.

### 1.3. CS expression upon starvation at the RNA level.

RNA was prepared from cells grown in HL-5 medium (vegetative stage) or after 4, 8 or 15 hours of starvation in PDF, respectively.

Therefor 10⁷ cells were lysed in 50mM Tris pH 8.5, 0.1% SDS, 1mM vanadyl complex and extracted 3 times with phenol-chloroform 1:1.

15 µg of RNA were treated with glyoxal and subjected to electrophoresis on a 0.8% agarose gel. The samples were transferred to Genescreen plus filters with a vacugene apparatus. Anti-sense RNA probes were generated by inserting DNA fragments onto the vector pGEM-1, and carrying out Sp6-RNA polymerase reactions. The filters were hybridized for 48 hours at 55°C in a solution containing 50% formamide, 5XSSC (1XSSC=0.15M NaCl, 15mM sodium citrate), 0.2% bovine serum albumin, 0.2% Ficoll, 0.2% polyvinylpyrrolidone, 25mM Na₂-HPO₄, 25mM NaH₂PO₄, 0.2% SDS, 1mM EDTA, 250 µg/ml denatured DNA and 500 µg/ml yeast RNA. The filters were washed 5X15 min. in 0.1XSSC, 0.12% SDS at 65°C.

On the Northern blots, an RNA species of 1.4 kb was detected in pEDII-CS transfected cell, whereas no signal was detected in pVEII transfected cells. The amount of steady-state CS RNA increases after 4 hours of starvation, followed by a decrease in expression after 15 hours.

### 1.4. CS expression upon starvation at the protein level.

Proteins were prepared from the same aliquots used in example 1.3. for the analysis of CS expression upon starvation at the protein level.

Cells were lysed in 1 X Laemmli's buffer at 100°C for 5 min. and proteins were separated on a 10% SDS-PAGE. Proteins were electrotransferred onto a nitrocellulose filter. 0.2 mg/ml of the anti-NANP monoclonal antibody was added to the filter for an overnight incubation at room temperature. ¹²⁵I-protein A was used to reveal the anti-NANP reaction.

The Western blots showed no CS expression in pVEII cells and a maximal expression in pEDII-CS cells after 4 hours of starvation. A single band of 62 kDA molecular mass was detected. No other crossreacting species was observed. This induction of the expression follows the profile expected for Discoidin I gene regulation.

### 1.5. Recognition of the NANP epitope on the CS protein.

To confirm recognition of the NANP epitope present on the CS protein, the binding of an anti-NANP monoclonal antibody to the Dd expressed CS protein was competed with a NANP₅₀ peptide. Cells expressing the CS polypeptide were lysed, proteins separated by SDS-PAGE and transferred onto a nitrocellulose filter. Before incubation, the anti-NANP₅₀ monoclonal antibody was incubated with different amount of a NANP₅₀ peptide which correspond to the number of NANP₅₀ repeats present on the CS protein. A tenth equimolar amount of NANP₅₀ peptide was sufficient to observe a significant reduction in the binding of the monoclonal antibody to the CS protein.

### 1.6. Recognition of different Plasmodium falciparum CS epitopes by monoclonal antibodies.

Different monoclonal antibodies against CS proteins or synthetic peptides were used in the immunoblot assay. Dd cells were lysed, proteins separated by SDS-PAGE and transferred onto a nitrocellulose filter. Filter strips were incubated with different monoclonal antibodies from various categories (SP3B4, SP3.E9, CT3.3, CT3.1, SP3.H3, SP3.E6, SP3.C6). Recognition of epitopes was visible only with the expected antibodies. In no cases, a specific signal was observed in cells expressing pVEII vector only.

### 1.7. Purification of CS protein produced in Dictyostelium discoideum.

At the amino acids level, the CS protein contains a carboxy terminal hydrophobic segment which could play a role as a signal for the addition of a glycosyl-phosphatidylinositol anchor (GPI). This C-terminal peptide segment or a posttranslationally added GPI anchor should confer a hydrophobic nature to the CS protein thus allowing its partitioning in Triton X-114.

Proteins were extracted from the cells with 1% of Triton X-114 and separated into aqueous and detergent phases. The detergent soluble proteins were analyzed by Western blot. At least 10 times more proteins were present in the aqueous phase than in the TX-114 phase estimated by a Biorad Protein assay and also as judged by Ponceau staining. A strong signal is observed in the TX-114 phase of CS expression cells, even though a certain amount of protein still remains in the aqueous phase. No signal is found in the pVEII sample. Treatment with Triton X-114 therefore allows a first partial purification of the recombinant protein.

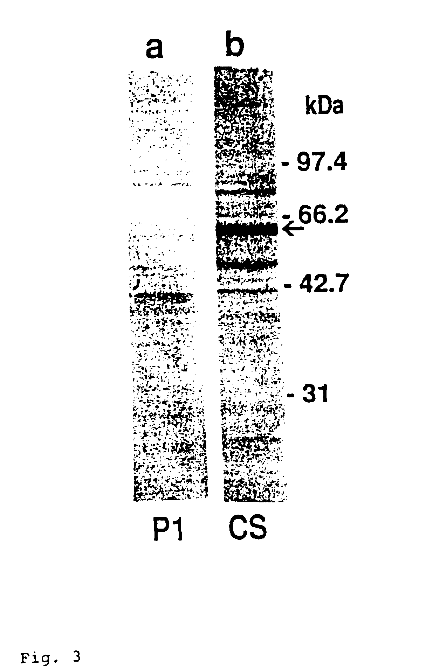

In order to further purify CS protein expressed in *Dictyostelium,* CS cells were pulse-labelled with [³⁵S]-methionine for 2 hours after an initial starvation period of two hours. Cells were removed by centrifugation, lysed in Crumpton lysis buffer and precleaned with protein A Sepharose. TX-114 soluble proteins were loaded on an affinity column containing anti-NANP antibody crosslinked to Sepharose. After elution from the column in the presence of 0.1 M glycine (pH 2.5) methionine-labelled protein with an apparent molecular weight of 62kDa was detected by SDS-PAGE and fluorography. The same experiment performed on pVEII containing cells revealed only minor bands of different molecular weights (Fig. 3).

### 1.8. Cell surface expression.

If correctly processed, the CS protein should be present on the cell surface. Analysis of fluorescent activated cell sorting using an anti-NANP antibody showed that the CS protein is expressed at the surface of *Dictyostelium discoideum* cells.

### 1.9. Terminal sequencing.

In order to determine whether the expressed CS protein was complete, CS protein was isolated, using Triton TX-114 phase separation and immunoaffinity chromatography, to sequence its N-terminus.

No sequence could be obtained from the complete protein, indicating a probable block at the amino terminus. When the purification was done in presence of fewer protease inhibitors, an amino acid sequence missing the first 25 amino acids was obtained. when only benzamidine was used up to 33 amino acids were absent from the N-terminus suggesting that. endogenous protease could attack the CS protein during its purification. As shown, this problem can be solved by using protease inhibitors and is limited to the first 20 amino acids.

### EXAMPLE 2

The following example teaches the expression of the circum sporozoite antigen CS of *Plasmodium falciparum* in *Dictyostelium discoideum* fused to a leader peptide from contact site A and under the control of an inducible promoter (Dd ras)

### 2.1. DNA-sequence of the Dd ras promoter

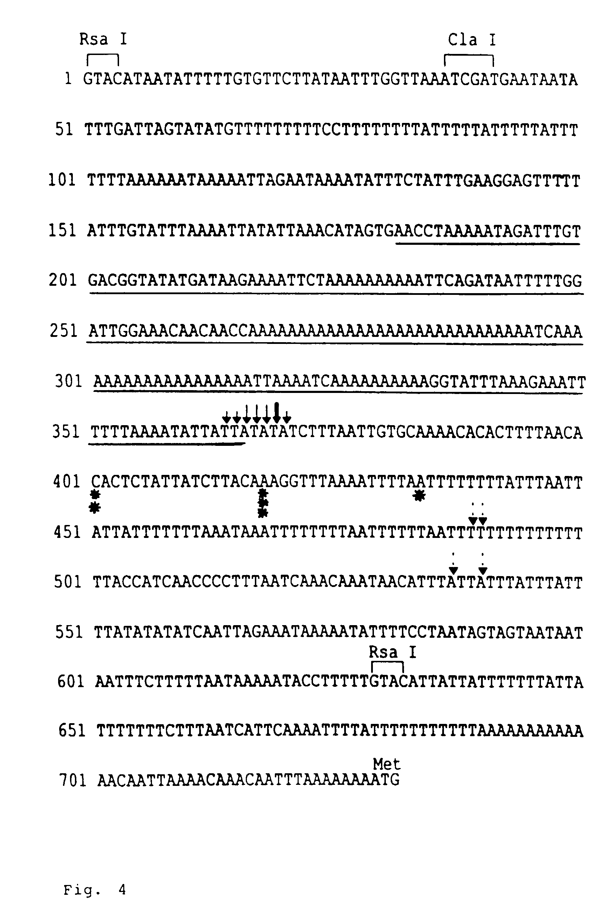

The DNA-sequence of the Dd ras promoter used in this example is identical to nucleotides 1 to 401 from Figure 4 (see also example 3)

### 2.2. Construction of pERI-CS vector

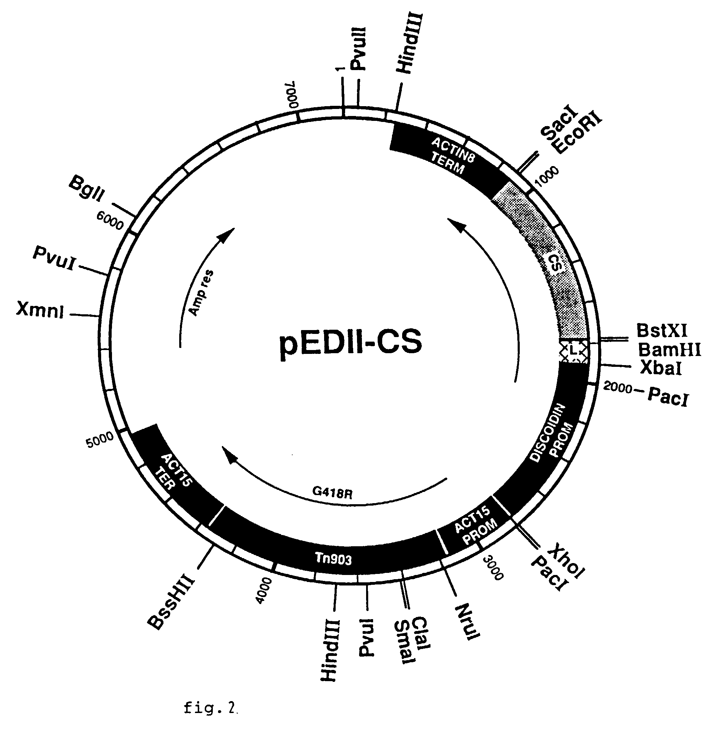

The Discoidin I promoter region of pEDII-CS was excised using PacI resriction digestion, leaving 68 nucleotides of transcribed but untranslated Discoidin I sequence, the AUG codon, the contact site A leader peptide and the CS coding region (see Figure 2). A Dd ras promoter fragment encompassing nucleotides 1 to 401 of Figure 4 was obtained by exonuclease III digestion and addition of *Bam*HI linkers. After blunt ending with T4-polymerase, this fragment was ligated in the above mentioned *Pac*I digested CS vector to obtain pERI-CS

### 2.3. Expression of the CS protein

The construct pERI-CS of example 2.2. was reintroduced into *Dictyostelium* and cells analyzed for CS expression by Western blot (see example 1.4.; results not shown). CS expression was very low in vegetative cells starved in suspension in PDF. An increase of about 100 fold was observed in the level of CS protein already after one hour of cAMP addition (0.2 mM). Two hours of induction further increased expression whereas the level of CS protein decreased after 16 hours of incubation.

### EXAMPLE 3

The following example teaches the expression of chloramphenicol acetyl transferase in *Dictyostelium discoideum* using an inducible expression vector.

### 3.1. DNA sequence of the Dd ras promoter.

The RsaI restriction fragment from the genomic clone of Dd ras was inserted into the SmaI site from pGEM1. Sequencing was performed using specific primers on double stranded DNA.

Fig. 4. shows the DNA sequence of the RsaI fragment. Arrows indicate transcription start sites. The thick arrow corresponds to the major transcript detected upon external cAMP addition. Dotted arrows indicate further RNA start sited used during normal Dd development. Met is the Dd ras protein start codon. The region essential for promoter function is underlined. * corresponds to the breakpoint of a 3' deletion providing a DNA fragment comprising nucleotides 1 to 434; ** indicates the breakpoint of a 3' deletion providing a DNA fragment comprising nucleotides 1 to 401; *** corresponds to the breakpoint of a 5' deletion providing a DNA fragment comprising nucleotides 418-630.

### 3.2. Construction of the CAT containing vector.

Parts of Dd ras promoter sequences were placed in pAV-CAT vector (May et al., Mol. Cell Biol. 9, 4653, 1989). The resulting constructs resemble pEDII-CS with CS and leader peptides replaced by a chloramphenicol acetyl transferase gene (CAT), Discoidin I promoter by Dd ras promoter and the G418 resistance gene under the control of the actin 15 promoter and terminator region placed in opposite orientation.

### 3.3. Expression of chloramphenicol acetyl transferase in Dictyostelium discoideum on the RNA level.

The constructs of example 2.2. were reintroduced in *Dictyostelium* and cells analyzed for CAT expression on the RNA level.

RNA was prepared from Dd cells transformed with a recombinant pAV-CAT vector containing parts of the RsaI fragment of the Dd ras promoter. RNA was isolated from cells starved for 7 hours. Half of the cells received cAMP (0.2mM) for 1 hour before RNA preparation. After separation on agarose gel (MES-glyoxal) and transfer to Genescreen, the RNA was probed with pAV-CAT whole vector labelled by nick translation.

CAT transcripts were detectable after cAMP induction with Dd ras promoter fragments containing at least the sequence underlined in Fig. 4. Further deletions abolished CAT RNA accumulation. Optimal CAT transcript accumulation occurred with a DNA containing sequence 1 to 401 (** in fig. 4).

### 3.4. Expression of chloramphenicol acetyl transferase in Dictyostelium discoideum at the enzyme level.

10⁷ Dd cells containing pAV-CAT vectors with nucleotides 1 to 434, 1 to 401 and 418 to 630 respectively were lysed by 3 cycles of freezing and thawing. After centrifugation at 12'000 rpm for 5 min. CAT activity in supernatant was assayed for 30 min. by described techniques (Gorman et al., Mol. Cell. Biol. 2, 1044, 1982). Due to large amounts of CAT activity in the extracts, only 1 µl out of 50 µl of supernatant was used to insure being in the linear phase of the reaction.

CAT activity assays showed not only the presence of a functional enzyme in Dd extracts, but also induction with the proper constructs.

## Claims

1. A recombinant DNA molecule for expression of a desired functional polypeptide or intermediate thereof in dictyostelid hosts, comprising:
a) a Dictyostelium discoideum homologous promoter region;
b) a Dictyostelium discoideum homologous terminator region;
c) a heterologous DNA sequence with a Dictyostelium discoideum homologous peptide sequence being capable of functioning as a leader peptide sequence positioned upstream thereof and in proper reading frame therewith,
wherein the heterologous DNA sequence encodes the desired functional polypeptide or intermediate thereof, said DNA sequence being positioned downstream from the promoter region and said terminator region being positioned downstream from the DNA sequence, wherein the Dictvostelium discoideum homologous leader peptide sequence is a sequence other than the D. discoideum PsA protein leader peptide sequence.

2. The recombinant DNA molecule as claimed in claim 1, **characterized in that** the leader peptide sequence is as contained in the XbaI to BamHI restriction fragment of the sequence depicted in Figure 1.

3. The recombinant DNA molecule according to claims 1 and 2, wherein the dictyostelid host is Dictyostelium discoideum.

4. The recombinant DNA molecule according to claims 1-3, wherein the homologous termination sequence is the actin 8 termination sequence of Dictyostelium discoideum.

5. The recombinant DNA molecule according to claims 1-4, wherein the promoter region is the Discoidin I-promoter of Dictyostelium discoideum.

6. The recombinant DNA molecule according to claims 1-4, wherein the promoter region is Dd ras promoter of Dictyostelium discoideum.

7. The recombinant DNA molecule according to claims 1-4, wherein the promoter region is composed of basepairs 1 to 401 of the Dd ras promoter as depicted in Figure 4.

8. The recombinant DNA molecule according to claims 1-7, wherein the heterologous DNA sequence encodes a parasite protein.

9. A recombinant DNA molecule according to claim 8, wherein the parasite protein is a human or animal parasite protein.

10. The recombinant DNA molecule according to claim 9, wherein the protein is a Plasmodium spp protein.

11. The recombinant DNA molecule according to claim 10, wherein the Plasmodium spp protein is a Plasmodium falciparum protein.

12. The recombinant DNA molecule according to claim 11, wherein the Plasmodium falciparum protein is the circumsporozoite antigen.

13. The recombinant DNA molecule according to claims 1-7, wherein the heterologous DNA sequence encodes a protein with enzymatic activity.

14. The expression plasmid pEDII-CS which is described in EXAMPLE 1.

15. The expression plasmid pERI-CS which is described in EXAMPLE 2.

16. A dictyostelid strain comprising a recombinant DNA molecule as claimed in claims 1-13 or an expression plasmid as claimed in claim 14 or 15.

17. A dictyostelid strain as claimed in claim 16 comprising a recombinant DNA molecule as claimed in claim 12 or the expression vector as claimed in claim 14 and expressing the circumsporozoite antigen from Plasmodium falciparum.

18. A dictyostelid strain as claimed in claim 16 comprising a recombinant DNA molecule as claimed in claim 13 and expressing an enzymatically active protein.

19. Method for producing a recombinant DNA molecule for expression of a desired polypeptide in dictyostelids, comprising:
a) linearising a DNA molecule containing Dictyostelium homologous promoter and terminator sequences to obtain a linear DNA molecule;
b) isolating a heterologous DNA sequence encoding the desired polypeptide;
c) ligating said linearized DNA molecule and said isolated DNA sequence to obtain a first recombinant DNA molecule;
d) synthesizing a leader peptide sequence as contained in the XbaI to BamHI restriction fragment of the sequence depicted in Fig.1;
e) linearizing the first recombinant plasmid of step c) to obtain linearized plasmid DNA; and
f) ligating said linear recombinant plasmid and said synthesized leader peptide sequence to obtain a second recombinant DNA molecule for transformation of a dictyostelid host.

20. Method for producing recombinant plasmids pEDII-CS as claimed in claim 14, wherein the DNA molecule containing Dictyostelium discoideum homologous promoter and terminator sequence is pVEII, the heterologous DNA sequence is the CS NF54 gene of Plasmodium falciparum, the first recombinant plasmid is pEDI-CS as claimed in claim 15 and the leader peptide is as contained in the XbaI to BamHI restriction fragment of the sequence depicted in Figure 1.

21. Method for producing recombinant polypeptides, which method comprises:
a) preparing a recombinant DNA molecule as claimed in claims 1-13 or an expression vector as claimed in claim 14 or 15 encoding a desired polypeptide sequence;
b) transforming a dictyostelid host cell culture with said DNA molecule or expression vector to obtain a recombinant dictyostelid host cell;
c) culturing said recombinant dictyostelid host cell under conditions permitting expression of the DNA sequence encoding the desired recombinant polypeptide; and
d) recovering said recombinant polypeptide.

22. The method as claimed in claim 21, wherein the desired polypeptide is a parasite protein.

23. The method as claimed in claim 22, wherein the desired polypeptide is a human or animal parasite protein.

24. The method as claimed in claim 23, wherein the desired parasite polypeptide is a Plasmodium spp protein.

25. The method as claimed in claim 24, wherein the Plasmodium spp protein is a Plasmodium falciparum protein.

26. The method as claimed in claim 25, wherein the Plasmodium falciparum protein is the circumsporozoite antigen.

27. The method as claimed in claim 21, wherein the desired polypeptide is a polypeptide having enzymatic activity.

28. Method as claimed in claims 21-27, wherein the dictyostelid host cell culture is Dictyostelium discoideum.

29. Method as claimed in claims 21-28, wherein the conditions permitting expression of the DNA sequence comprise starving the Dictyostelium cell culture.

30. Method for producing a recombinant DNA molecule for expression of a desired polypeptide having enzymatic activity in dictyostelids comprising:
a) linearizing pAV-CAT to obtain a linear DNA molecule;
b) isolating at least parts of the Dictyostelium discoideum Dd ras promoter;
c) ligating said linearized DNA molecule and said isolated promoter sequence to obtain a recombinant DNA molecule for transformation of a dictyostelid host;

31. Method for producing polypeptides having enzymatic activity, which method comprises:
a) preparing an expression vector according to the method of claim 30;
b) transforming a dictyostelid host cell culture with said expression vector to obtain a recombinant dictyostelid host cell;
c) culturing said recombinant dictyostelid host cell under starvation conditions so as to permit expression of the DNA sequence encoding the desired recombinant polypeptide having enzymatic activity after addition of cAMP; and
d) recovering said recombinant polypeptide having enzymatic activity.

## Patentansprüche

1. Rekombinantes DNA-Molekül zur Expression eines gewünschten funktionalen Polypeptids oder einer Zwischenverbindung hiervon in Dictyostelid-Wirten, umfassend:
a) eine Dictyostelium discoideum homologe Promotorregion;
b) eine Dictyostelium discoideum homologe Terminatorregion;
c) eine heterologe DNA-Sequenz, wobei eine Dictyostelium discoideum homologe Peptidsequenz, die in der Lage ist, als Leader-Peptidsequenz zu dienen, stromaufwärts davon positioniert ist und in einem geeigneten Leseraster liegt,
wobei die heterologe DNA-Sequenz das gewünschte funktionale Polypeptid oder eine Zwischenverbindung hiervon codiert, wobei die DNA-Sequenz stromabwärts der Promotorregion angeordnet ist und die Terminatorregion stromabwärts der DNA-Sequenz angeordnet ist, wobei die Dictyostelium discoideum homologe Leader-Peptidsequenz eine andere Sequenz ist, als die D. discoideum PsA Protein-Leader-Peptidsequenz.

2. Rekombinantes DNA-Molekül nach Anspruch 1, dadurch gekennzeichnet, daß die Leader-Peptidsequenz in dem XbaI bis BamHI Restriktionsfragment der in Figur 1 dargestellten Sequenz enthalten ist.

3. Rekombinantes DNA-Molekül nach den Ansprüchen 1 und 2, in dem der Dictyostelid-Wirt Dictyostelium discoideum ist.

4. Rekombinantes DNA-Molekül nach den Ansprüchen 1 bis 3, in dem die homologe Terminationssequenz die Aktin-8-Terminationssequenz von Dictyostelium discoideum ist.

5. Rekombinantes DNA-Molekül nach den Ansprüchen 1 bis 4, in dem die Promotorregion der Discoidin-I-Promotor von Dictyostelium discoideum ist.

6. Rekombinantes DNA-Molekül nach den Ansprüchen 1 bis 4, in dem die Promotorregion der Dd ras-Promotor von Dictyostelium discoideum ist.

7. Rekombinantes DNA-Molekül nach den Ansprüchen 1 bis 4, in dem die Promotorregion aus den Basenpaaren 1 bis 401 des Dd ras-Promotors, wie in Figur 4 dargestellt, zusammengesetzt ist.

8. Rekombinantes DNA-Molekül nach den Ansprüchen 1 bis 7, in dem die heterologe DNA-Sequenz ein parasitäres Protein codiert.

9. Rekombinantes DNA-Molekül nach Anspruch 8, in dem das parasitäre Protein ein menschliches oder tierisches parasitäres Protein ist.

10. Rekombinantes DNA-Molekül nach Anspruch 9, in dem das Protein ein Plasmodium spp.-Protein ist.

11. Rekombinantes DNA-Molekül nach Anspruch 10, in dem das Plasmodium spp.-Protein ein Plasmodium falciparum-Protein ist.

12. Rekombinantes DNA-Molekül nach Anspruch 11, in dem das Plasmodium falciparum-Protein das Circumsporozoit-Antigen ist.

13. Rekombinantes DNA-Molekül nach den Ansprüchen 1 bis 7, in dem die heterologe DNA-Sequenz ein Protein mit enzymatischer Aktivität codiert.

14. Expressionsplasmid pEDII-CS, das in BEISPIEL 1 beschrieben ist.

15. Expressionsplasmid pERI-CS, das in BEISPIEL 2 beschrieben ist.

16. Dictyostelid-Stamm, der ein rekombinantes DNA-Molekül nach den Ansprüchen 1 bis 13 oder ein Expressionsplasmid nach Anspruch 14 oder 15 umfaßt.

17. Dictyostelid-Stamm nach Anspruch 16, der ein rekombinantes DNA-Molekül nach Anspruch 12 oder den Expressionsvektor nach Anspruch 14 umfaßt und das Circumsporozoit-Antigen von Plasmodium falciparum exprimiert.

18. Dictyostelid-Stamm nach Anspruch 16, der ein rekombinantes DNA-Molekül nach Anspruch 13 umfaßt und ein enzymatisch aktives Protein exprimiert.

19. Verfahren zur Herstellung eines rekombinanten DNA-Moleküls zur Expression eines gewünschten Polypeptids in Dictyosteliden, umfassend:
a) Linearisieren eines DNA-Moleküls, das Dictyostelium homologe Promotor- und Terminatorsequenzen enthält, um ein lineares DNA-Molekül zu erhalten;
b) Isolieren einer heterologen DNA-Sequenz, die das gewünschte Polypeptid codiert;
c) Ligieren des linearisierten DNA-Moleküls und der isolierten DNA-Sequenz, um ein erstes rekombinantes DNA-Molekül zu erhalten;
d) Synthetisieren einer Leader-Peptidsequenz, wie sie in dem XbaI bis BamHI-Restriktionsfragment der in Fig. 1 dargestellten Sequenz enthalten ist;
e) Linearisieren des ersten rekombinanten Plasmids von Schritt c), um linearisierte Plasmid-DNA zu erhalten; und
f) Ligieren des linearen rekombinanten Plasmids und der synthetisierten Leader-Peptidsequenz, um ein zweites rekombinantes DNA-Molekül zur Transformation eines Dictyostelid-Wirts zu erhalten.

20. Verfahren zur Herstellung rekombinanter pEDII-CS Plasmide nach Anspruch 14, worin das die Dictyostelium discoideum homologe Promotor- und Terminator-Sequenz enthaltende DNA-Molekül pVEII ist, die heterologe DNA-Sequenz das CS NF54-Gen von Plasmodium falciparum ist, das erste rekombinante Plasmid pEDI-CS nach Anspruch 15 ist, und das Leader-Peptid in dem XbaI bis BamHI Restriktionsfragment der in Figur 1 dargestellten Sequenz enthalten ist.

21. Verfahren zur Herstellung rekombinanter Polypeptide, welches umfaßt:
a) Herstellen eines rekombinanten DNA-Moleküls nach den Ansprüchen 1 bis 13 oder eines Expressionsvektors nach Anspruch 14 oder 15, das eine gewünschte Polypeptidsequenz codiert;
b) Transformieren einer Dictyostelid-Wirtszellenkultur mit dem DNA-Molekül oder dem Expressionsvektor, um eine rekombinante Dictyostelid-Wirtszelle zu erhalten;
c) Kultivieren der rekombinanten Dictyostelid-Wirtszelle unter Bedingungen, die die Expression der DNA-Sequenz, die das gewünschte rekombinante Polypeptid codiert, ermöglichen; und d) Gewinnen des rekombinanten Polypeptids.

22. Verfahren nach Anspruch 21, in dem das gewünschte Polypeptid ein parasitäres Protein ist.

23. Verfahren nach Anspruch 22, in dem das gewünschte Polypeptid ein menschliches oder tierisches parasitäres Protein ist.

24. Verfahren nach Anspruch 23, in dem das gewünschte parasitäre Polypeptid ein Plasmodium spp.-Protein ist.

25. Verfahren nach Anspruch 24, in dem das Plasmodium spp.-Protein ein Plasmodium falciparum-Protein ist.

26. Verfahren nach Anspruch 25, in dem das Plasmodium falciparum-Protein das Circumsporozoit-Antigen ist.

27. Verfahren nach Anspruch 21, in dem das gewünschte Polypeptid ein Polypeptid mit enzymatischer Aktivität ist.

28. Verfahren nach den Ansprüchen 21 bis 27, in denen die Dictyostelid-Wirtszellenkultur Dictyostelium discoideum ist.

29. Verfahren nach den Anspüchen 21 bis 28, in dem die Bedingungen, die die Expression der DNA-Sequenz ermöglichen, das Hungernlassen der Dictyostelium-Zellkultur umfassen.

30. Verfahren zur Herstellung eines rekombinanten DNA-Moleküls zur Expression eines gewünschten Polypeptids mit enzymatischer Aktivität in Dictyosteliden, umfassend:
a) Linearisieren von pAV-CAT, um ein lineares DNA-Molekül zu erhalten;
b) Isolieren von zumindest Teilen des Dictyostelium discoideum Dd ras-Promotors;
c) Ligieren des linearisierten DNA-Moleküls und der isolierten Promotorsequenz, um ein rekombinantes DNA-Molekül zur Transformation eines Dictyostelid-Wirts zu erhalten.

31. Verfahren zur Herstellung von Polypeptiden mit enzymatischer Aktivität, das umfaßt:
a) Herstellen eines Expressionsvektors nach dem Verfahren von Anspruch 30;
b) Transformieren einer Dictyostelid-Wirtszellenkultur mit dem Expressionsvektor, um eine rekombinante Dictyostelid-Wirtszelle zu erhalten;
c) Kultivieren der rekombinanten Dictyostelid-Wirtszelle unter Hunger-Bedingungen, um die Expression der DNA-Sequenz, die das gewünschte, nach Zugabe von cAMP enzymatische Aktivität besitzende rekombinante Polypeptid codiert, zu ermöglichen; und
d) Gewinnen des rekombinanten Polypeptids mit enzymatischer Aktivität.

## Revendications

1. Molécule d'ADN recombinant destinée à l'expression d'un polypeptide fonctionnel désiré ou d'un de ses intermédiaire chez des hôtes dictyostélides, comprenant :
a) une région promotrice homologue de Dictyostelium discoideum ;
b) une région terminale homologue de Dictyostelium discoideum ;
c) une séquence d'ADN hétérologue avec une séquence peptidique homologue de Dictyostelium discoideum étant capable de fonctionner comme une séquence peptidique leader positionnée en amont de celle-ci et dans un cadre de lecture correct avec celle-ci,
dans laquelle la séquence d'ADN hétérologue code pour le polypeptide fonctionnel désiré ou son intermédiaire, ladite séquence d'ADN se trouvant en aval de la région promotrice et ladite région terminale se trouvant en aval de la séquence d'ADN, dans laquelle la séquence peptidique leader homologue de Dictyostelium discoideum est une séquence autre que la séquence peptidique leader de la protéine PsA de D. discoideum.

2. Molécule d'ADN recombinant selon la revendication 1, caractérisée en ce que la séquence peptidique leader est telle qu'elle est contenue dans le fragment de restriction de XbaI à BamHI de la séquence représentée sur la figure 1.

3. Molécule d'ADN recombinant selon les revendications 1 et 2, dans laquelle l'hôte dictyostélide est Dictyostelium discoideum.

4. Molécule d'ADN recombinant selon les revendications 1 à 3, dans laquelle la séquence terminale homologue est la séquence terminale de l'actine 8 de Dictyostelium discoideum.

5. Molécule d'ADN recombinant selon les revendications 1 à 4, dans laquelle la région promotrice est le promoteur de la Discoïdine I de Dictyostelium discoideum.

6. Molécule d'ADN recombinant selon les revendications 1 à 4, dans laquelle la région promotrice est le promoteur Dd ras du Dictyostelium discoideum.

7. Molécule d'ADN recombinant selon les revendications 1 à 4, dans laquelle la région promotrice se compose des paires de bases 1 à 401 du promoteur Dd ras tel qu'il est représenté sur la figure 4.

8. Molécule d'ADN recombinant selon les revendications 1 à 7, dans laquelle la séquence d'ADN hétérologue code pour une protéine de parasite.

9. Molécule d'ADN recombinant selon la revendication 8, dans laquelle la protéine de parasite est une protéine de parasite humain ou animal.

10. Molécule d'ADN recombinant selon la revendication 9, dans laquelle la protéine est une protéine ssp de Plasmodium.

11. Molécule d'ADN recombinant selon la revendication 10, dans laquelle la protéine ssp de Plasmodium est une protéine de Plasmodium falciparum.

12. Molécule d'ADN recombinant selon la revendication 11, dans laquelle la protéine de Plasmodium falciparum est l'antigène de circumsporozoïte.

13. Molécule d'ADN recombinant selon les revendications 1 à 7, dans laquelle la séquence d'ADN hétérologue code pour une protéine ayant une activité enzymatique.

14. Le plasmide d'expression pEDII-CS décrit dans l'exemple 1.

15. Le plasmide d'expression pERI-CS décrit dans l'exemple 2.

16. Souche de dictyostélide comprenant une molécule d'ADN recombinant selon les revendications 1 à 13 ou un plasmide d'expression selon les revendications 14 ou 15.

17. Souche de dictyostélide selon la revendication 16, comprenant une molécule d'ADN recombinant selon la revendication 12 ou le vecteur d'expression selon la revendication 14 et exprimant l'antigène de circumsporozoïte à partir de Plasmodium falciparum.

18. Souche de dictyostélide selon la revendication 16, comprenant une molécule d'ADN recombinant selon la revendication 13 et exprimant une protéine enzymatiquement active.

19. Procédé de production d'une molécule d'ADN recombinant destinée à l'expression d'un polypeptide désiré chez des dictyostélides, comprenant les étapes consistant à :
a) linéariser une molécule d'ADN contenant les séquences promotrice et terminale homologues de Dictyostelium pour obtenir une molécule d'ADN linéaire ;
b) isoler une séquence d'ADN hétérologue codant pour le polypeptide désiré ;
c) ligaturer la molécule d'ADN linéarisée et la séquence d'ADN isolée pour obtenir une première molécule d'ADN recombinant ;
d) synthétiser une séquence peptidique leader contenue dans le fragment de restriction de XbaI à BamHI de la séquence représentée sur la figure 1 ;
e) linéariser le premier plasmide recombinant de l'étape c) pour obtenir de l'ADN plasmidique linéarisé ; et
f) ligaturer le plasmide recombinant linéaire et la séquence peptidique leader de synthèse pour obtenir une deuxième molécule d'ADN recombinant destinée à la transformation d'un hôte dictyostélide.

20. Procédé de production de plasmides recombinants pEDII-CS selon la revendication 14, dans lequel la molécule d'ADN contenant les séquences promotrice et terminale homologues de Dictyostelium discoideum est pVEII, la séquence d'ADN hétérologue est le gène CS NF54 de Plasmodium falciparum, le premier plasmide recombinant est pEDI-CS selon la revendication 15 et le peptide leader est contenu dans le fragment de restriction de XbaI à BamHI de la séquence représentée sur la figure 1.

21. Procédé de production de polypeptides recombinants, le procédé comprenant :
a) préparer une molécule d'ADN recombinant selon les revendications 1 à 13 ou un vecteur d'expression selon la revendication 14 ou 15 codant une séquence de polypeptide désirée ;
b) transformer une culture de cellules hôtes de dictyostélides avec la molécule d'ADN ou le vecteur d'expression pour obtenir une cellule hôte de dictyostélide recombinante ;
c) cultiver la cellule hôte de dictyostélide recombinante dans des conditions permettant l'expression de la séquence d'ADN codant pour le polypeptide recombinant désiré ; et
d) recueillir le polypeptide recombinant.

22. Procédé selon la revendication 21, dans lequel le polypeptide désiré est une protéine de parasite.

23. Procédé selon la revendication 22, dans lequel le polypeptide désiré est une protéine de parasite humain ou animal.

24. Procédé selon la revendication 23, dans lequel le polypeptide de parasite désirée est une protéine spp de Plasmodium.

25. Procédé selon la revendication 24, dans lequel la protéine spp de Plasmodium est une protéine de Plasmodium falciparum.

26. Procédé selon la revendication 25, dans lequel la protéine de Plasmodium falciparum est l'antigène de circumsporozoïte.

27. Procédé selon la revendication 21, dans lequel le polypeptide désiré est un polypeptide ayant une activité enzymatique.

28. Procédé selon les revendications 21 à 27, dans lequel la culture de cellules hôtes de dictyostélides est formée de Dictyostelium discoideum.

29. Procédé selon les revendications 21 à 28, dans lequel les conditions permettant l'expression de la séquence d'ADN comprennent la privation de la culture de cellules de Dictyostelium.

30. Procédé de production d'une molécule d'ADN recombinant destinée à l'expression d'un polypeptide désiré ayant une activité enzymatique chez les dictyostélides, comprenant :
a) linéariser pAV-CAT pour obtenir une molécule d'ADN linéaire ;
b) isoler au moins certaines parties du promoteur Dd ras de Dictyostelium discoideum ;
c) ligaturer la molécule d'ADN linéarisée et la séquence promotrice isolée pour obtenir une molécule d'ADN recombinant destinée à la transformation d'un hôte dictyostélide.

31. Procédé de production de polypeptides ayant une activité enzymatique, le procédé comprenant :
a) préparer un vecteur d'expression selon le procédé de la revendication 30 ;
b) transformer une culture de cellules hôtes de dictyostélides avec le vecteur d'expression pour obtenir une cellule hôte de dictyostélide recombinante ;
c) cultiver la cellule hôte de dictyostélide recombinante dans des conditions de privation de façon à permettre l'expression de la séquence d'ADN codant le polypeptide recombinant désiré ayant une activité enzymatique après ajout d'AMPc ; et
d) recueillir le polypeptide recombinant ayant une activité enzymatique.
